# EUROPÄISCHE PATENTSCHRIFT

(11) **EP 4 272 638 B1**
(45) Veröffentlichungstag und Bekanntmachung des Hinweises auf die Patenterteilung: **10.06.2026**
(21) Anmeldenummer: 23020207.9
(22) Anmeldetag: 03.05.2023
(51) Int. Cl.: A61B 5/08, A61B 5/083, A61B 5/097

(54) **SYSTEM ZUR ERFASSUNG VON ATEMGASBESTANDTEILEN**
SYSTEM FOR SENSING RESPIRATORY GAS COMPONENTS
SYSTÈME DE DÉTECTION DE COMPOSANTS DE GAZ RESPIRATOIRE

(30) Priorität: 04.05.2022 DE 102022111059
(43) Veröffentlichungstag der Anmeldung: 08.11.2023
(73) Patentinhaber: Cortex Biophysik GmbH, 04356 Leipzig (DE)
(72) Erfinder: Günther, Andreas, 04158 Leipzig (DE); Siepmann, Markus, 45473 Mülheim an der Ruhr (DE)
(74) Vertreter: Dr. Völger, Wolfgang

(56) Entgegenhaltungen:
- WO-A1-01/47417
- WO-A1-2019/074922
- DE-A1- 102014 204 625
- DE-A1- 102016 013 906
- DE-A1- 102021 111 431
- DE-B3- 102014 004 765
- US-A1- 2020 022 618
- US-A1- 2021 072 225

## Beschreibung

Die vorliegende Erfindung betrifft ein System zur Erfassung von Atemgasbestandteilen, das insbesondere in der Spiroergometrie einsetzbar ist.

Bei dem diagnostischen Verfahren der Spiroergometrie (auch als Ergospirometrie oder Ergospirographie bezeichnet) werden durch die Messung von Atemgasen während einer körperlichen Belastung die Reaktionen von Herz, Kreislauf, Atmung, Stoffwechsel und/oder die kardiopulmonale Leistungsfähigkeit qualitativ und quantitativ untersucht.

Spiroergometrie-Geräte sind daher als hochpräzise medizinische Messinstrumente ausgelegt, woraus hohe Anforderungen an die Messgenauigkeit des jeweiligen Gerätes resultieren. Aufgrund von inhärenten Faktoren (z.B. Alterung, Verbrauch) und äußeren Einflüssen (z.B. Temperaturschwankungen) kommt es zu einer Veränderung der Kennlinien der in den Spiroergometrie-Geräten eingesetzten Sensoren. Diese können somit die geforderte Messgenauigkeit über einen längeren Zeitraum nicht einhalten. Deshalb muss in regelmäßigen Abständen die Messabweichung (Kalibration) bestimmt und das Messsignal entsprechend justiert werden. Damit trägt die Kalibrierung/Justierung wesentlich dazu bei, dass die Messergebnisse von Spiroergometrie-Geräten den Anforderungen aus etablierten Standards der Spiroergometrie und den Marktanforderungen gerecht werden.

Gewöhnlich kommen in Spiroergometrie-Geräten zwei Sensoren zur Gasanalyse zum Einsatz, zum einen ein Sauerstoffsensor (O₂-Sensor) und zum anderen ein Kohlenstoffdioxidsensor (CO₂-Sensor).

Die Sensorkennlinie des Sauerstoffsensors wird stark von der Temperatur beeinflusst, da die chemische Reaktion durch thermische Einflüsse schneller oder langsamer abläuft. Außerdem verbrauchen sich die chemischen Reaktanten ähnlich wie in einer Batterie. Dadurch wird mit Dauer der Nutzung des Sauerstoffsensors das Ausgangssignal immer schwächer. Eine echte Langzeitstabilität ist in herkömmlichen Sauerstoffsensoren selten gegeben.

Der Kohlenstoffdioxidsensor nutzt beispielsweise das Nah-Infrarot-Absorptionsspektrum. Kohlenstoffdioxid-Moleküle absorbieren Infrarotlicht einer ganz bestimmten Wellenlänge. Der Kohlenstoffdioxidsensor besteht aus einer Infrarotquelle und einem Detektor für genau diese Wellenlänge. Die Sensorkennlinie wird durch von außen eindringende Wärmestrahlung und die Alterung der Infrarotlichtquelle bestimmt.

Bei beiden Kennlinien von Sauerstoffsensor und Kohlenstoffdioxidsensor verändern aufgrund der genannten Einflüsse sowohl ihren Anstieg als auch ihre Verschiebung.

Um die Kennlinie der beiden Sensoren sowohl im Nullpunkt zu verschieben als auch den Anstieg dieser Kennlinie anzupassen, werden zwei exakte Referenzmesspunkte benötigt. Die Zweipunkt-Kalibrierung (bzw. Zweipunktjustierung) ist aktuell Stand der Technik. Ein Referenzmesspunkt ist in der Regel ein Prüfgas (bzw. Kalibriergas), welches in seiner Zusammensetzung die Exspirationsluft des Menschen imitiert (15 Vol.-% O₂, 5 Vol.-% CO₂, Rest N₂). Dieses Prüfgas wird technisch mit ausreichender Genauigkeit hergestellt. Als zweiter Referenzmesspunkt wird die Umgebungsluft herangezogen, welche der Inspirationsluft entspricht. Bei Frischluft geht man von einer festen Zusammensetzung mit 20,93 Vol.-% O₂ und 0,035 Vol.-% CO₂ (Rest hauptsächlich N₂) aus. In geschlossenen Räumen und Laboren kann aber durch die anwesenden Personen die Zusammensetzung der Umgebungsluft teils stark variieren.

Hinzu kommt, dass in klinischen Einrichtungen und Laboren nicht immer die Möglichkeit besteht, über ein Fenster den Raum zu durchlüften. Teilweise sind feste Lüftungsanlagen installiert, die dies übernehmen sollen. Wie gut die Frischluftversorgung mit solchen Anlagen funktioniert, ist unbekannt. Da in diesen genannten Fällen die genaue Zusammensetzung des zweiten Referenzmesspunktes unbekannt ist, wird die Zusammensetzung von Frischluft angenommen und somit ein Messfehler in Kauf genommen.

Dieser Messfehler kann mehrere Prozent betragen, abhängig davon, wie weit die Gaszusammensetzung im Raum von den zugrunde gelegten Frischluftwerten abweicht. In geschlossenen, schlecht durchlüfteten Räumen sind Kohlenstoffdioxidanteile von 0,4 Vol.-% und höher nicht ungewöhnlich. In Ausnahmefällen können sogar Werte über 1 Vol.-% auftreten.

Angesichts der vorstehend dargelegten Probleme besteht ein großer Bedarf daran, die auftretenden Messfehler, welche bei einer Kalibrierung an Umgebungsluft durch eine unbekannte Umgebungsluftzusammensetzung entstehen, zumindest zu minimieren, wenn nicht sogar vollständig zu verhindern.

Dieser Bedarf kann bislang mit den herkömmlichen Ansätzen nicht befriedigend erfüllt werden. So wurde beispielsweise vorgeschlagen, einen CO₂-Absorber (z.B. sog. Atemkalk) im Gerät zu verbauen, der das CO₂ der Umgebungsluft absorbiert und somit einen Referenzpunkt mit 0 Vol.-% CO₂ erzeugt. Alternativ gibt es den Ansatz, ein zweites Prüfgas mit einer zu Frischluft vergleichbaren Zusammensetzung als austauschbares Kalibriergasflasche im Gerät zu verwenden. Beide Varianten sind jedoch mit einem Materialbedarf verbunden und machen die Kalibration/Justierung handlungsaufwendiger, ohne den vorstehend geschilderten Bedarf tatsächlich zu erfüllen.

Wieder andere Ansätze sehen O₂-Sensoren vor, um den tatsächlichen O2 Gehalt der Umgebungsluft zu ermitteln. Allerdings ergibt sich dabei das Problem, dass Sauerstoffsensoren mit einer entsprechenden Empfindlichkeit und Messgenauigkeit keine Langzeitstabilität aufweisen und selbst regelmäßig kalibriert oder ausgetauscht werden müssen. Die Kalibrierung müsste dann z. B. wieder aufwändig mit zusätzlichen Prüfgasen erfolgen, wodurch kein Mehrwert zum aktuellen Stand der Technik geschaffen wird.

Der vorliegen Erfindung liegt daher die Aufgabe zugrunde, ein neuartiges System zur Erfassung von Atemgasbestandteilen bereitzustellen, welches die auftretenden Messfehler bei Kalibrierung an Umgebungsluft minimiert und gleichzeitig einfach und effizient in der Handhabung ist. Weiterhin hat die Erfindung das Ziel, den Nutzer über den aktuellen CO₂-Gehalt im Raum zu informieren, um im Vorfeld von durchgeführten Leistungsmessungen bereits entsprechende Gegenmaßnahmen einzuleiten (Raumbelüftung).

Diese Aufgabe wird in der vorliegenden Erfindung durch ein System (100) zur Erfassung von Atemgasbestandteilen gelöst, umfassend
- einen Atemgaseinlass (101),
- eine mit dem Atemgaseinlass (101) verbundene Messzelle (103) mit zumindest einem Atemgassensor,
- einen Gasauslass (105) stromabwärts der Messzelle (103),
- eine mit der Messzelle (103) verbundene Datenverarbeitungseinheit,
- eine Energieversorgung (107),
dadurch gekennzeichnet, dass das System (100) einen zusätzlichen Sensor (109) zur Messung zumindest des Kohlendioxidanteils der Umgebungsluft des Systems (100) aufweist.

Mit "Atemgaseinlass" wird in der vorliegenden Erfindung ein Anschluss verstanden, an den für die Verwendung des Systems (100) ein Schlauch angeschlossen wird, der zu einer Atemmaske des Probanden führt.

Bei der "Messzelle" (103) handelt es sich erfindungsgemäß um eine Anordnung von Atemgassensoren (meist ein Sauerstoff und ein Kohlendioxidsensor) und einer Messeinheit zur Erfassung der Durchflussrate (z. B. ein Differenzdrucksensor), die pneumatisch in Reihe angeschlossen sind und vom, aus der Atemmaske abgesaugten Atemgas, durchflossen werden. Alle Elemente der "Messzelle" (103) sind mit einer Datenverarbeitungseinheit, insbesondere in Form eines Mikrocontrollers verbunden.

Der "Gasauslass" stellt die Abführung der gemessenen Atemgase aus dem System (100) sicher.

Die Energieversorgung (107) ist erfindungsgemäß beispielsweise ein Netzanschluss und/oder eine Batterieeinheit.

Kennzeichnend für das erfindungsgemäße System (100) ist der zusätzliche Sensor (109) zur Messung zumindest des Kohlendioxidanteils der Umgebungsluft des Systems (100). Durch die Messung der tatsächlich in der Umgebungsluft vorhanden CO₂-Konzentration (Kohlendioxidanteil), anstelle der Verwendung theoretischer Standardkonzentrationswerte (O₂: 20,93 Vol.-%; CO₂: 0,035 Vol.-%), ist eine genaue und nicht nur auf theoretischen Werten basierende Kalibrierung der Atemgassensoren der Messzelle (103) möglich, wodurch die Nachteile des Standes der Technik überwunden werden. Dieser Sensor kann folglich als eigener "Referenzsensor" zur Kalibrierung auf der Grundlage der tatsächlichen in der Umgebungsluft vorliegenden CO₂-Konzentration angesehen werden.

Der zusätzliche Sensor (109) ist insbesondere dazu ausgelegt nur den Kohlendioxidanteil der Umgebungsluft des Systems (100) zu messen. Dieser kann daher als eigener "Referenzsensor" zur Kalibrierung der Atemgassensoren der Messzelle (103) auf der Grundlage der ermittelten CO₂-Konzentration angesehen werden.

Die vorliegende Erfindung weist ganz allgemein die Vorteile auf, dass mit dem erfindungsgemäßen System (100) eine genaue Messung der tatsächlichen CO₂-Konzentration der Umgebungsluft vorgenommen werden kann, um bei dem System (100) den Fehler bei der Kalibrierung der Atemgassensoren der Messzelle (103) minimieren zu können.

Für diese erfindungsgemäße Lösung sind keine Verbrauchsmaterialien (CO₂-Absorber, Prüfgas, etc.) notwendig, wie sie im Stand der Technik benötigt werden. Ferner ist der erfindungsgemäß verwendete zusätzliche Sensor (109) von kleiner Bauart und lässt sich damit auch in ein kompaktes und tragbares Gerät integrieren. Diese Merkmalskombination führt zudem dazu, dass eine automatische Zweipunkt-Kalibrierung ermöglicht wird.

Darüber hinaus ist es möglich, den Nutzern / Probanden Informationen über den aktuellen CO₂-Gehalt im Raum zur Verfügung zu stellen. So können bereits im Vorfeld von durchzuführenden Leistungsmessungen entsprechende Gegenmaßnahmen wie eine Raumbelüftung vorgenommen werden.

Eine Weiterbildung sieht vor, dass der zusätzliche Sensor (109) zur optimierten Kalibrierung der Atemgassensoren der Messzelle (103) auf der Grundlage des tatsächlichen Kohlendioxidanteils der Umgebungsluft des Systems (100) ausgelegt ist.

Anders als im bekannten Stand der Technik wird erfindungsgemäß für die Umgebungsluft nicht die Standardzusammensetzung mit einer festen CO₂-Konzentration (meist 0,035 Vol.-%) angenommen, sondern die tatsächliche CO₂-Konzentration der Umgebungsluft ermittelt und der Kalibrierung des Atemgassensors zugrunde gelegt. Hiermit werden die systematischen Messfehler, wie sie vorstehende für den Stand der Technik beschrieben wurden, verhindert. Nach der Kalibrierung des Atemgassensors mit der tatsächlichen CO₂-Konzentration kann eine korrekte und zuverlässige Messung der Atemgasbestandteile erfolgen.

Es hat sich für die Umsetzung der vorliegenden Erfindung als vorteilhaft herausgestellt, wenn der zusätzliche Sensor (109) zur Messung zumindest des Kohlendioxidanteils ein kalibrierarmer Sensor mit hoher Langzeitstabilität ist. Der für die Erfindung verwendete Sensor hat ein Kalibrationsintervall von 5 Jahren und ist ansonsten weitgehend wartungsfrei. Da diese Kalibrierung allerdings auch mit Aufwand für den Nutzer verbunden wäre (Einsatz von Kalibrationsgas etc.) kann der erfindungsgemäß verwendete Sensor nach 5 Jahren einfach durch das Wechselfach ausgetauscht werden.

Im Gegensatz zu den bekannten Sauerstoffsensoren, die meistens nicht langzeitstabil sind und regelmäßig justiert oder ausgetauscht werden müssen, wird in der vorliegenden Erfindung ein zumindest in Bezug auf die Wechseleinheit (111) mit integriertem zusätzlichem Sensor (109) zur Messung zumindest des Kohlendioxidanteils wartungsarmes System (100) zur Verfügung gestellt, das ohne Probleme auch von fachlich nicht geschulten Nutzern / Probanden sicher und sinnvoll eingesetzt werden kann.

Eine andere erfindungsgemäße Ausführungsform des Systems (100) sieht vor, dass der zusätzliche Sensor (109) zur Messung nur des Kohlendioxidanteils mindestens eine Messgenauigkeit von ± 50 ppm + 3 % aufweist.

Bei der Anwendung des erfindungsgemäßen Systems (100) für die Spiroergometrie haben bereits geringe Änderungen der Gaskonzentrationen der Umgebungsluft (wenige ppm) deutlichen Einfluss auf die Messergebnisse, wenn mit theoretischen Standartkonzentrationen kalibriert wird. Aus dem aktuellen Stand der Technik sind jedoch keine Sauerstoffsensoren bekannt, die derart kleine Änderungen der O₂-Konzentration in einem Raum und/oder über einen langen Zeitraum stabil messen können, ohne selbst kalibriert zu werden. Theoretisch für diesen Zweck einsetzbare Sauerstoffsensoren für die Erfassung der Umgebungsluft haben eine Baugröße, die sich nicht in ein kompaktes und tragbares Gerät integrieren lässt. Diese Sauerstoffsensoren sind zudem wartungsintensiv. Der Fachmann wird daher für Sauerstoff-Umgebungssensors sehr hohe technische Hürden erkennen.

In der vorliegenden Erfindung wurde herausgearbeitet, dass eine Umrechnung von im Stand der Technik gemessenen O₂-Konzentrationen in CO₂-Konzentrationen grundsätzlich möglich ist, um dann einen Atemgassensor zu kalibrieren. Denn die von einem Menschen aufgenommene Menge an Sauerstoff (VO₂) wird in ein äquivalentes Volumen an Kohlendioxid (VCO₂) umgewandelt. Fällt also beispielsweise der O₂-Anteil in einem Raum um 0,5 Vol.-%, steigt damit der CO₂-Anteil um 0,5 Vol.-%.

Mit dem erfindungsgemäß verwendeten zusätzlichen Sensor (109) kann somit nicht nur der CO₂-Atemgassensor in der Messzelle (103) mit dem tatsächlichen CO₂-Gehalt der Umgebungsluft kalibriert werden, sondern es ist auch eine Kalibrierung des O₂-Atemgassensors der Messzelle (103) durch Rückrechnung des gemessenen CO₂-Gehalts der Umgebungsluft auf den tatsächlichen O₂-Gehalt der Umgebungsluft.

Eine ganz exakte äquivalente Umrechnung ist nicht möglich, da durch verschiedene Stoffwechselprozesse im menschlichen Körper (z.B. Mischung aus Fett- & Kohlenhydrat-Verbrennung) keine völlig äquivalente Umwandlung stattfindet. Der dadurch entstehende Fehler liegt dennoch unter dem Fehler, der ohne Berücksichtigung einer Raumluftänderung eintreten würde.

In einer bevorzugten Ausführungsform des erfindungsgemäßen Systems (100) ist der zusätzliche Sensor (109) zur Messung zumindest des Kohlendioxidanteils in einer von außen zugänglichen Wechseleinheit (111) angeordnet, deren Positionierung am erfindungsgemäßen System (100) darüber hinaus dazu ausgelegt ist, den zusätzlichen Sensor (109) von Atemgasbestandteilen abzuschirmen.

Bezogen auf den Stand der Technik werden bei CO₂-Sensoren zur Raumluftüberwachung Kalibrierintervalle von bis zu fünf Jahren angegeben. Innerhalb dieses Zeitraums sichern die Hersteller zu, dass die CO₂-Sensoren die angegebene Messgenauigkeit (z.B. von ± 50 ppm + 3 % vom Messwert) einhalten. Eine solche Langzeitstabilität ist von herkömmlichen Sauerstoffsensoren nicht bekannt. Da das erfindungsgemäße System (100) auf eine Lebenszeit von mindestens zehn Jahren ausgelegt wurde, muss der CO₂-Sensor entweder nachkalibrierbar oder austauschbar sein. Der Aufwand einer Re-Kalibration durch einen Nutzer (Verbraucher bzw. NichtFachmann) ist aufgrund der offenen Bauweise der meisten der verfügbaren CO₂-Sensoren sehr hoch und birgt die große Problematik einer Fehlkalibration. Dieser Mehraufwand soll erfindungsgemäß vermieden werden. Aus diesem Grund wird der CO₂-Sensor (109) in der vorliegenden Erfindung für den Nutzer austauschbar gestaltet durch die von außen zugängliche Wechseleinheit (111) im System (100).

Um die von außen zugängliche Wechseleinheit (111) vor Ausatemluft abzuschirmen, befindet sich diese insbesondere auf der Rückseite des Systems, also auf der dem Nutzer abgewandten Seite, damit durch die normale Atmung des Nutzers der als Referenz herangezogene CO₂-Sensor (109) (und damit die Kalibrierung) nicht beeinflusst werden. Zudem werden durch Anbringen auf der Rückseite die Wechseleinheit (111) und der CO₂-Sensor (109) vor Einflüssen von ungewollten Berührungen durch die übliche Handhabung des Systems (100) geschützt.

In einer vorteilhaften Weiterbildung des erfindungsgemäßen Systems (100) ist ferner eine Einrichtung (113) zum aktiven Gasaustausch mit der Umgebungsluft vorgesehen. Diese Einrichtung (113) kann als pneumatisches Schlauchsystem mit einem Magnetventil als Kernstück ausgeführt sein. Hiermit sind auch schnellere Änderungen des Kohlendioxidanteils der Umgebungsluft präzise zu erfassen.

Diese Weiterbildung dient dazu, Änderungen der CO₂-Konzentration in einem Raum schnell und präzise erfassen zu können. Passive Belüftungen, die auf reiner Konvektion beruhen, würden nur einen langsamen Austausch mit der Umgebungsluft ermöglichen und daher schnelle Änderungen der Raumluftqualität nur verzögert detektieren. Hierzu wird der erfindungsgemäße zusätzliche Sensor (109) intensiv mit Umgebungsluft durchflutet, zum einen durch das Vorsehen einer Vielzahl von Belüftungslöchern im Gehäuse der Wechseleinheit (111), zum anderen durch die Einrichtung (113), durch welche bei der Kalibration die zu messende Umgebungsluft unmittelbar durch die Wechseleinheit (111) abgesaugt wird.

Es hat sich für eine möglichst praktische und einfache Nutzung des erfindungsgemäßen Systems (100) als vorteilhaft herausgestellt, wenn dieses ferner eine vollautomatische Kalibriereinheit umfasst. Die vorstehend beschriebene für die CO₂-Messung notwendige aktive Gasabsaugung der Umgebungsluft kann dadurch zu einer vollautomatischen Kalibrierung erweitert werden, dass eine Prüfgasflasche an das System (100) anschließbar ist. Während der Zweipunkt-Kalibrierung wird dabei durch die Einrichtung (113) zwischen Umgebungsluft und Prüfgas umgestellt.

In einer anderen bevorzugten Ausführungsform des erfindungsgemäßen Systems (100) weist die Energieversorgung (107) Akkuzellen (1071) in zumindest einem von außen zugänglichen Wechselfach (115) auf.

Dabei können die Akkuzellen (1071) redundant angeordnet sein, so dass entleerte / verbrauchte Akkuzellen (1071) ausgetauscht werden können während gleichzeitig noch geladene Akkuzellen (1071) die Versorgung mit Energie sicherstellen, so dass der Betrieb des erfindungsgemäßen Systems (100) nicht unterbrochen werden muss.

Das erfindungsgemäße System (100) ist in einer speziellen Weiterbildung mit einer mobilen Vorrichtung (1) und einer stationären Einheit (3) zweiteilig ausgebildet,
wobei die mobile Vorrichtung (1) den Atemgaseinlass (101), die Messzelle (103) mit zumindest einem Atemgassensor, den Gasauslass (105) und die Datenverarbeitungseinheit zusammen mit einer eigenen Energieeinheit (117) umfasst,
wobei die stationäre Vorrichtung (3) zur reversiblen Aufnahme der mobilen Vorrichtung (1) ausgelegt ist.

Diese spezielle Weiterbildung der vorliegenden Erfindung vereint die Vorteile einer mobilen Vorrichtung (1) in Punkto Variabilität und Beweglichkeit mit den Vorteilen einer stationären Einheit (3), welche mit Merkmalen versehen ist, die in einer mobilen Vorrichtung (1) nicht untergebracht werden können, wenn diese für den mobilen Einsatz durch Probanden komfortabel und handhabbar sein soll.

Dabei ist der für das erfindungsgemäße System (100) kennzeichnende zusätzliche Sensor (109) zur Messung zumindest des Kohlendioxidanteils der Umgebungsluft in der stationären Vorrichtung (3) angeordnet, da diese normalerweise in geschlossenen Räumen aufgestellt ist. Demgegenüber wird die mobile Vorrichtung (1) in der Regel im Freien verwendet, wo von Frischluft als Umgebungsluft mit den vorstehend genannten Gehalten an Sauerstoff und Kohlenstoffdioxid ausgegangen werden kann.

Durch Auslagerung verschiedener, für die mobile Messung nicht erforderlicher Merkmale in die stationäre Vorrichtung (3), kann die mobile Vorrichtung (1) sehr klein und komfortabel für den mobilen Einsatz gestaltet werden.

Für die spezielle Weiterbildung der vorliegenden Erfindung hat es sich als vorteilhaft herausgestellt, wenn die mobile Vorrichtung (1) und die stationäre Einheit (3) jeweils eine mechanische Rastverbindung (119a, 119b) zur lösbaren kraftschlüssigen Verbindung miteinander und eine Datenschnittstelle (121) zum Anschluss aneinander aufweisen.

Damit wird zunächst sichergestellt, dass die mobile Vorrichtung (1) nicht versehentlich von der stationären Einheit (3) entfernt wird oder herunterfällt. Ferner wird durch die mechanische Rastverbindung (119a, 119b) der einwandfreie Anschluss der Datenschnittstelle (121) gewährleistet.

Um die mobile Vorrichtung (1) der speziellen Weiterbildung der vorliegenden Erfindung auch bei Bewegung des Nutzers (bspw. Sport im Freien) problemlos verwenden zu können, ist besonders bevorzugt die mobile Vorrichtung (1) ferner mit einer mechanischen Rastverbindung (119c) zur lösbaren kraftschlüssigen Verbindung mit einem Körpertragesystem (5) versehen. Diese mechanische Rastverbindung (119c) kann vorzugsweise mit der mechanischen Rastverbindung (119b) zur Verbindung mit der stationären Einheit (3) identisch sein.

Das erfindungsgemäße System (100) nach der speziellen Weiterbildung der vorliegenden Erfindung zeichnet sich zudem dadurch aus, dass die mobile Vorrichtung (1) eine Abmessung von 15,6 cm × 16,2 cm × 4,8 cm nicht überschreitet. Durch diese Abmessung ist die mobile Vorrichtung (1) auch bei Bewegungen des Nutzers nicht störend und kann, insbesondere mit dem passenden Körpertragesystem (5), weitgehend ohne Einschränkungen der Beweglichkeit verwendet werden. Mobile Vorrichtungen für die Spiroergometrie mit vergleichbaren technischen Kenndaten sind in diesen geringen Abmessungen aus dem Stand der Technik nicht bekannt.

Die geringen Abmessungen der mobilen Vorrichtung (1) ergeben sich insbesondere aus der erfindungsgemäßen Aufteilung der erforderlichen Merkmale abhängig vom Einsatzzweck in geschlossenen Räumen oder im Freien. Auf diese Weise kann das zweiteilig ausgebildete System (100) dieser speziellen Ausführungsform nicht nur alle vorstehend beschriebenen Vorteile realisieren, sondern stellt auch eine maximale Variabilität in der Anwendung zur Verfügung.

In einem zweiten Aspekt der vorliegenden Erfindung ist das erfindungsgemäße System (100), wie es vorstehend beschrieben wurde, für die Verwendung in der Spiroergometrie, in der Spirometrie und in der Ruheumsatzmessung ausgelegt.

Die Erfindung wird durch die beigefügten Ansprüche definiert.

Es zeigen:
- Fig. 1: eine grafische Darstellung des erfindungsgemäßen Systems 100 in einer bevorzugten Ausführungsform,
- Fig. 2: eine grafische Darstellung des erfindungsgemäßen Systems 100 der Figur 1 in einer seitlichen Rückansicht,
- Fig. 3: eine grafische Detaildarstellung des erfindungsgemäßen Systems 100 der Figur 1,
- Fig. 4: ein Blockdiagramm des erfindungsgemäßen Systems 100 in einer bevorzugten Ausführungsform.

In den Figuren werden alle gleichen Bauteile mit den gleichen Bezugszeichen benannt, aus Gründen der Übersichtlichkeit sind aber nicht unbedingt in allen Darstellungen alle Bezugszeichen eingefügt.

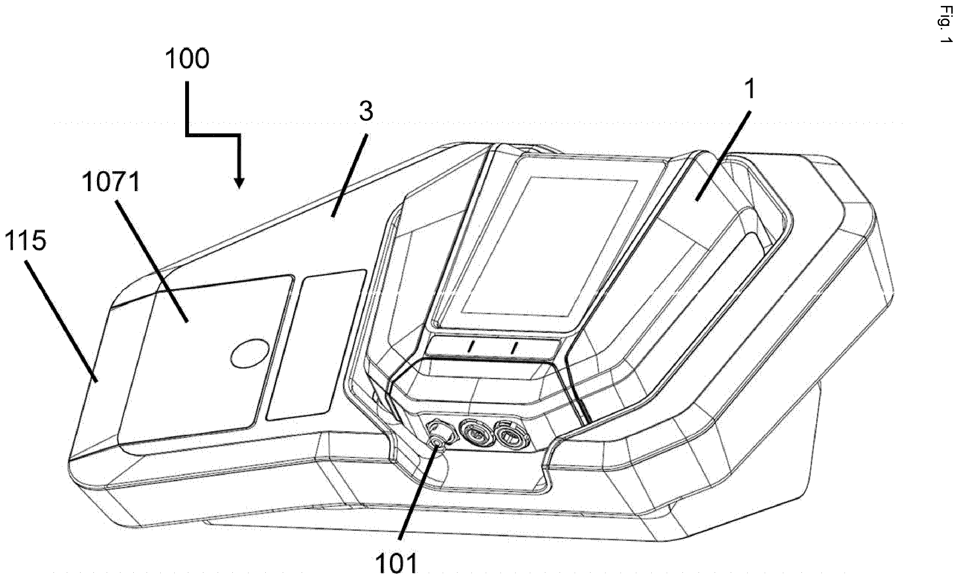

In Figur 1 wird eine grafische Darstellung des erfindungsgemäßen Systems 100 als Ganzes in einer bevorzugten Ausführungsform gezeigt. Diese bevorzugte Ausführungsform beruht auf der zweiteiligen Ausführung mit mobiler Vorrichtung 1 und stationärer Einheit 3. Die mobile Vorrichtung 1 ist als mobiles Messgerät ausgelegt, das aus dem System 100 für Messungen im Feldeinsatz herausgenommen werden kann. Die Figur 1 zeigt ferner die Anordnung des von außen zugänglichen Wechselfache 115, in welchem die Akkuzellen 1071 aufgenommen sind. An der Vorderseite des Systems 100, konkret an der mobilen Vorrichtung 1 verschiedene Anschlüsse, darunter insbesondere der Atemgaseinlass 101.

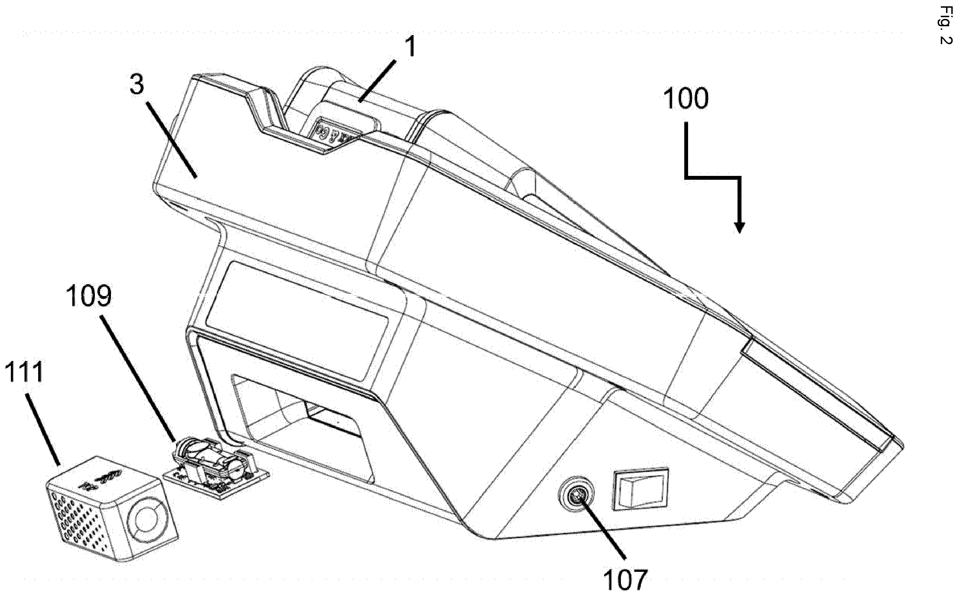

Figur 2 zeigt eine grafische Darstellung des erfindungsgemäßen Systems 100 der Figur 1 in einer von der Seite gesehenen Rückansicht, aus der deutlich wird, wie der CO₂-Sensor 109 und die Wechseleinheit 111 auf der Rückseite angeordnet sind, um einerseits nicht oder kaum mit dem Ausatemgas in Kontakt zu kommen und andererseits versehentlich Berührungen bei der Bedienung durch einen Nutzer zu vermeiden. Ferner ist der Anschluss für die Energieversorgung 107 gekennzeichnet.

Die von außen zugängliche Wechseleinheit 111 wird in die nicht mit einem Bezugszeichen versehene Aufnahmemulde eingebracht und nimmt ihrerseits in ihrem Inneren den CO₂-Sensor 109 auf. Gut zu erkennen sind die Öffnungen in der Wechseleinheit 111 für den Eintritt der Umgebungsluft, die gleichzeitig eine anderweitige Kontamination weitgehend unterbinden.

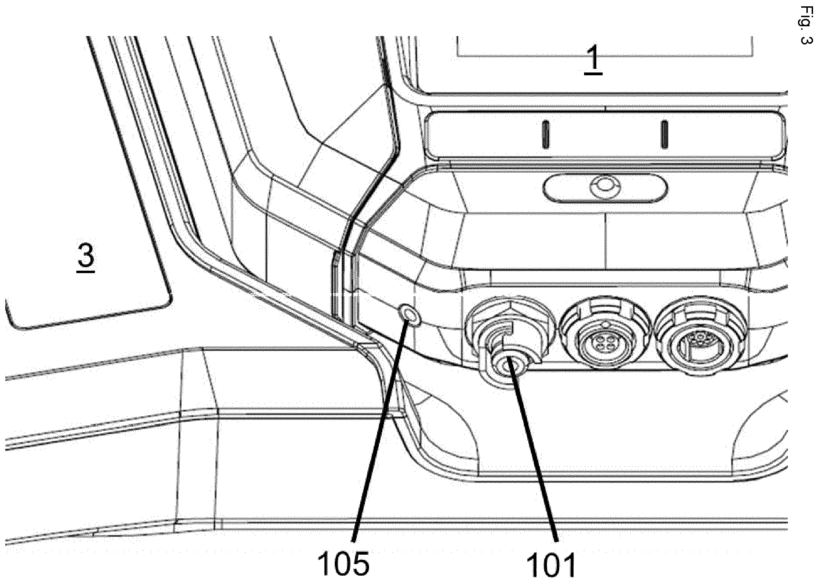

In Figur 3 wird eine grafische Detaildarstellung gezeigt, aus welcher die verschiedenen Anschlüsse zu erkennen sind, darunter insbesondere wieder der Atemgaseinlass 101 sowie der Gasauslass 105 stromabwärts der hier nicht dargestellten Messzelle 103.

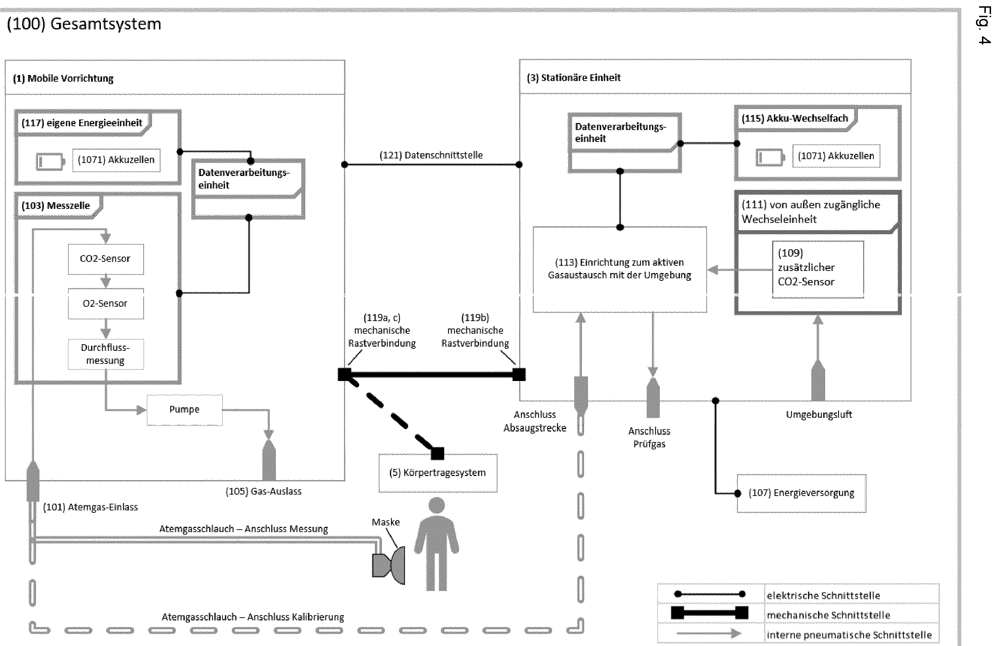

Figur 4 zeigt schließlich ein Blockdiagramm des erfindungsgemäßen Systems 100 in der hier beschriebenen bevorzugten Ausführungsform. Die einzelnen Komponenten wurden vorstehend schon beschrieben oder sind dem Blockdiagramm selbsterklärend zu entnehmen. In der gewählten Ansicht sind insbesondere die einzelnen Leitungssysteme und Verbindungen dargestellt, die sich auf das pneumatische System mit internen pneumatischen Schnittstellen, auf die elektrischen Verbindungen mit den elektrischen Schnittstellen und auf die mechanischen Verbindungen mit den mechanischen Schnittstellen aufteilen.

### Bezugszeichenliste

- 1: mobile Vorrichtung
- 100: System zur Erfassung von Atemgasbestandteilen
- 101: Atemgaseinlass
- 103: Messzelle
- 105: Gasauslass
- 107: Energieversorgung
- 1071: Akkuzellen
- 109: zusätzlicher Sensor / CO₂-Sensor
- 111: Wechseleinheit
- 113: Einrichtung zum aktiven Gasaustausch
- 115: Wechselfach
- 117: Energieeinheit
- 119a 119b: Rastverbindung
- 121: Datenschnittstelle
- 3: stationäre Einheit
- 5: Körpertragesystem

## Patentansprüche

1. System (100) zur Erfassung von Atemgasbestandteilen, umfassend
- einen Atemgaseinlass (101),
- eine mit dem Atemgaseinlass (101) verbundene Messzelle (103) mit zumindest einem Atemgassensor,
- einen Gasauslass (105) stromabwärts der Messzelle (103),
- eine mit der Messzelle (103) verbundene Datenverarbeitungseinheit,
- eine Energieversorgung (107),
**dadurch gekennzeichnet, dass** das System (100) einen zusätzlichen Sensor (109) zur Messung zumindest des Kohlendioxidanteils der Umgebungsluft des Systems (100) aufweist,
wobei der zusätzliche Sensor (109) zur Messung nur des Kohlendioxidanteils der Umgebungsluft des Systems (100) ausgelegt ist und
wobei der zusätzliche Sensor (109) zur optimierten Kalibrierung der Atemgassensoren der Messzelle (103) auf der Grundlage des Kohlendioxidanteils der Umgebungsluft des Systems (100) ausgelegt ist.

2. System (100) nach Anspruch 1, wobei der zusätzliche Sensor (109) zur Messung zumindest des Kohlendioxidanteils ein kalibrierarmer Sensor mit hoher Langzeitstabilität ist.

3. System (100) nach einem der Ansprüche 1 oder 2, wobei der zusätzliche Sensor (109) zur Messung nur des Kohlendioxidanteils mindestens eine Messgenauigkeit von ± 50 ppm + 3 % aufweist.

4. System (100) nach einem der Ansprüche 1 bis 3, wobei der zusätzliche Sensor (109) zur Messung zumindest des Kohlendioxidanteils in einer von außen zugänglichen Wechseleinheit (111) angeordnet ist, deren Positionierung am erfindungsgemäßen System (100) dazu ausgelegt ist, den zusätzlichen Sensor (109) von Atemgasbestandteilen abzuschirmen.

5. System (100) nach einem der Ansprüche 1 bis 4, wobei ferner eine Einrichtung (113) zum aktiven Gasaustausch mit der Umgebungsluft vorgesehen ist.

6. System (100) nach einem der Ansprüche 1 bis 5, ferner umfassend eine vollautomatische Kalibriereinheit.

7. System (100) nach einem der Ansprüche 1 bis 6, wobei die Energieversorgung (107) Akkuzellen (1071) in zumindest einem von außen zugänglichen Wechselfach (115) aufweist.

8. System (100) nach einem der Ansprüche 1 bis 7, wobei das System (100) mit einer mobilen Vorrichtung (1) und einer stationären Einheit (3) zweiteilig ausgebildet ist, wobei die mobile Vorrichtung (1) den Atemgaseinlass (101), die Messzelle (103) mit zumindest einem Atemgassensor, den Gasauslass (105) und die Datenverarbeitungseinheit zusammen mit einer eigenen Energieeinheit (117) umfasst,
wobei stationäre Vorrichtung (3) zur reversiblen Aufnahme der mobilen Vorrichtung (1) ausgelegt ist.

9. System (100) nach Anspruch 8, wobei die mobile Vorrichtung (1) und die stationäre Einheit (3) jeweils eine mechanische Rastverbindung (119a, 119b) zur lösbaren kraftschlüssigen Verbindung miteinander und eine Datenschnittstelle (121) zum Anschluss aneinander aufweisen.

10. System (100) nach Anspruch 8 oder 9, wobei die mobile Vorrichtung (1) ferner eine mechanische Rastverbindung (119c) zur lösbaren kraftschlüssigen Verbindung mit einem Körpertragesystem (5) aufweist.

11. System (100) nach einem der Ansprüche 8 bis 10, wobei die mobile Vorrichtung (1) eine Abmessung von 15,6 cm × 16,2 cm × 4,8 cm nicht überschreitet.

12. Verwendung des Systems (100) nach einem der Ansprüche 1 bis 11 für die Spiroergometrie.

## Claims

1. System (100) for detecting respiratory gas components, comprising
- a respiratory gas inlet (101),
- a measuring cell (103) connected to the respiratory gas inlet (101) and having at least one respiratory gas sensor,
- a gas outlet (105) downstream of the measuring cell (103),
- a data processing unit connected to the measuring cell (103),
- an energy supply (107),
**characterised in that** the system (100) has an additional sensor (109) for measuring at least the carbon dioxide content of the ambient air of the system (100),
wherein the additional sensor (109) is designed to measure only the carbon dioxide content of the ambient air of the system (100) and
wherein the additional sensor (109) is designed for optimised calibration of the respiratory gas sensors of the measuring cell (103) on the basis of the carbon dioxide content of the ambient air of the system (100).

2. System (100) according to claim 1, wherein the additional sensor (109) for measuring at least the carbon dioxide content is a low-calibration sensor with high long-term stability.

3. System (100) according to one of claims 1 or 2, wherein the additional sensor (109) for measuring only the carbon dioxide content has a measurement accuracy of at least ± 50 ppm + 3%.

4. System (100) according to one of claims 1 to 3, wherein the additional sensor (109) for measuring at least the carbon dioxide content is arranged in an externally accessible exchangeable unit (111) whose positioning on the system (100) according to the invention is designed to shield the additional sensor (109) from respiratory gas components.

5. System (100) according to one of claims 1 to 4, wherein an active gas exchange facility (113) with the ambient air is also provided.

6. System (100) according to one of claims 1 to 5, further comprising a fully automatic calibration unit.

7. System (100) according to one of claims 1 to 6, wherein the energy supply (107) comprises battery cells (1071) in at least one externally accessible exchangeable compartment (115).

8. System (100) according to one of claims 1 to 7, wherein the system (100) is designed in two parts with a mobile device (1) and a stationary unit (3),
wherein the mobile device (1) comprises the respiratory gas inlet (101), the measuring cell (103) with at least one respiratory gas sensor, the gas outlet (105) and the data processing unit together with its own energy unit (117),
wherein the stationary unit (3) is designed for the reversible accommodation of the mobile device (1).

9. System (100) according to claim 8, wherein the mobile device (1) and the stationary unit (3) each have a mechanical latching connection (119a, 119b) for releasable positive connection to each other and a data interface (121) for connection to each other.

10. System (100) according to claim 8 or 9, wherein the mobile device (1) further comprises a mechanical latching connection (119c) for releasable friction-locked connection to a body carrier system (5).

11. System (100) according to any one of claims 8 to 10, wherein the mobile device (1) does not exceed dimensions of 15.6 cm × 16.2 cm × 4.8 cm.

12. Use of the system (100) according to any one of claims 1 to 11 for spiroergometry.

## Revendications

1. Système (100) pour la détection de composants du gaz respiratoire, comprenant:
- une entrée de gaz respiratoire (101),
- une cellule de mesure (103) reliée à l'entrée de gaz respiratoire (101) avec au moins un capteur de gaz respiratoire,
- une sortie de gaz (105) en aval de la cellule de mesure (103),
- une unité de traitement de données reliée à la cellule de mesure (103),
- une alimentation en énergie (107),
**caractérisé en ce que** le système (100) comporte un capteur supplémentaire (109) pour mesurer au moins la teneur en dioxyde de carbone de l'air ambiant du système (100),
le capteur supplémentaire (109) étant conçu pour mesurer uniquement la teneur en dioxyde de carbone de l'air ambiant du système (100) et
le capteur supplémentaire (109) étant conçu pour l'étalonnage optimisé des capteurs de gaz respiratoire de la cellule de mesure (103) sur la base de la teneur en dioxyde de carbone de l'air ambiant du système (100).

2. Système (100) selon la revendication 1, dans lequel le capteur supplémentaire (109) pour mesurer au moins la teneur en dioxyde de carbone est un capteur nécessitant peu d'étalonnage, présentant une forte stabilité à long terme.

3. Système (100) selon l'une des revendications 1 ou 2, dans lequel le capteur supplémentaire (109) pour mesurer uniquement la teneur en dioxyde de carbone présente une précision de mesure d'au moins ± 50 ppm + 3 %.

4. Système (100) selon l'une des revendications 1 à 3, dans lequel le capteur supplémentaire (109) pour mesurer au moins la teneur en dioxyde de carbone est disposé dans une unité interchangeable (111) accessible de l'extérieur, dont le positionnement sur le système (100) selon l'invention est conçu pour protéger le capteur supplémentaire (109) des composants du gaz respiratoire.

5. Système (100) selon l'une des revendications 1 à 4, dans lequel est en outre prévu un dispositif (113) d'échange actif de gaz avec l'air ambiant.

6. Système (100) selon l'une des revendications 1 à 5, comprenant en outre une unité d'étalonnage entièrement automatique.

7. Système (100) selon l'une des revendications 1 à 6, dans lequel l'alimentation en énergie (107) comporte des cellules d'accumulateur (1071) dans au moins un compartiment interchangeable (115) accessible de l'extérieur.

8. Système (100) selon l'une des revendications 1 à 7, dans lequel le système (100) est réalisé en deux parties avec un dispositif mobile (1) et une unité stationnaire (3), le dispositif mobile (1) comprenant l'entrée de gaz respiratoire (101), la cellule de mesure (103) avec au moins un capteur de gaz respiratoire, la sortie de gaz (105) et l'unité de traitement de données, conjointement avec une unité d'énergie propre (117),
l'unité stationnaire (3) étant conçue pour recevoir de manière réversible le dispositif mobile (1).

9. Système (100) selon la revendication 8, dans lequel le dispositif mobile (1) et l'unité stationnaire (3) comportent chacun une liaison d'encliquetage mécanique (119a, 119b) pour une liaison démontable par adhérence l'une avec l'autre et une interface de données (121) pour le raccordement l'une à l'autre.

10. Système (100) selon la revendication 8 ou 9, dans lequel le dispositif mobile (1) comporte en outre une liaison d'encliquetage mécanique (119c) pour une liaison démontable par adhérence avec un système de portage corporel (5).

11. Système (100) selon l'une des revendications 8 à 10, dans lequel le dispositif mobile (1) ne dépasse pas une dimension de 15,6 cm x 16,2 cm x 4,8 cm.

12. Utilisation du système (100) selon l'une des revendications 1 à 11 pour la spiroergométrie.
